# EUROPEAN PATENT SPECIFICATION

(11) **EP 2 535 023 B1**
(45) Date of publication and mention of the grant of the patent: **11.10.2017**
(21) Application number: 12004509.1
(22) Date of filing: 14.06.2012
(51) Int. Cl.: A61F 2/28, A61F 2/30, A61F 2/44, A61F 2/46

(54) **Intervertebral implant**
Bandscheibenimplantat
Implant intervertébral

(30) Priority: 14.06.2011 EP 11169884; 14.06.2011 US 201161496801 P
(43) Date of publication of application: 19.12.2012
(73) Proprietor: Biedermann Technologies GmbH & Co. KG, 78166 Donaueschingen (DE)
(72) Inventor: Biedermann, Lutz, 78048 VS-Villingen (DE); Matthis, Wilfried, 79367 Weisweil (DE); Schwarzenbach, Othmar, 3612 Steffisburg (CH)
(74) Representative: Prüfer & Partner mbB Patentanwälte · Rechtsanwälte

(56) References cited:
- WO-A1-03/026538
- WO-A1-2011/108950
- US-A1- 2007 162 129
- US-A1- 2007 213 826
- US-A1- 2008 027 544
- US-A1- 2008 091 211
- US-A1- 2008 221 694
- US-A1- 2010 114 105

## Description

The invention relates to an intervertebral implant that has a top surface configured to engage a first vertebral body, a bottom surface configured to engage a second vertebral body and a sidewall connecting the top surface and the bottom surface, a hollow space defined by the sidewall and an elongate opening extending through the sidewall into the space. The implant further comprises a rotatable insert that is held within the space by a spring force. The insert has an engagement portion configured to engage with a tool through the opening.

An intervertebral implant configured to engage with an insertion device for inserting between first and second vertebral members is known from US 7,935,148. The intervertebral implant has an opening including an elongated shape that extends through the sidewall. A connection member including a receptacle is contained within the sidewall. The insertion device has a first end configured to connect with the connection member of the implant body. The first end is selectively positionable between orientations to provide for the connection.

Another intervertebral implant is known from US 2010/0094422 A1. The implant has a support body and a rotatable insert therein. An installation instrument is also disclosed for removable attachment to the implant and engagement with the rotatable insert to selectively permit rotation between the insert and the support body. Document US 2008/00912211 A1 discloses a spinal implant with an upper face, a lower face and a sidewall defining a hollow space. The sidewall is provided with an elongate opening along the circumferential direction; however, the opening does not extend through the sidewall into the hollow space. A rotatable insert within the opening is secured by a snap ring.

Document WO 2011/108950 A1 discloses a prosthesis comprising a body and a positioner, whereby a situating element projects from an external surface of the positioner and engages a positioning hole of the body, so as to limit mobility of the positioner inside the body.

Document US 2008/0221694 A1 shows an interbody spacer with a top face, a bottom face and a sidewall defining a hollow space. The side wall is provided with an elongate opening extending through the side wall into the space.

Document US 2007/0213826 A1 discloses an intervertebral spacer having an upper face, a lower face and a sidewall defining a hollow space. The side wall is also provided with an elongated opening along the circumferential direction without extending through the side wall into the space.

It is the object of the invention to provide an intervertebral implant that is simplified in terms of design and use and in view of the possibilities that are available for final positioning of the implant between the vertebral bodies.

The object is solved by an intervertebral implant according to claim 1. Further developments are given in the dependent claims.

The intervertebral implant has a compact design. The insertion procedure of the implant is simple and safe, since the connection between the implant and an insertion tool can be easily fixed and loosened. When the connection between a tool and the implant is loosened, the implant can be moved into a desired position by rotating it around the insert. During rotation, the intervertebral implant is safely held by an insertion device and is prevented from disconnecting from the tool.

The design of the intervertebral implant allows that a great portion of the hollow interior space of the implant is available for fusion.

Existing intervertebral implants could be modified and upgraded with the insert.

Further features and advantages of the invention will become apparent from the description of the accompanying drawings. In the drawings:
- Fig. 1: shows a perspective view of an embodiment of an intervertebral implant and a tool for inserting the intervertebral implant
- Fig. 2: shows an enlarged view of a portion of Fig. 1.
- Fig. 3: shows an exploded perspective view of the enlarged portion of Fig. 2.
- Fig. 4: shows a perspective view of an insert provided for the intervertebral implant.
- Fig. 5: shows another perspective view of the insert of Fig. 4.
- Fig. 6: shows a perspective view from above of the intervertebral implant in a step before inserting the insert.
- Fig. 7: shows a perspective view from above of the intervertebral implant with mounted insert.
- Fig. 8a): shows a cross-sectional view through the implant with the insert in a first position limited by a first stop.
- Fig. 8b): shows a cross-sectional view of the intervertebral implant with the insert in a second position limited by a second stop.
- Figs. 9a) to 9d): show schematic cross-sectional views of steps of engaging a tool with the intervertebral implant and rotating the implant.
- Figs. 10a) to 10f): schematically show steps of inserting and positioning the intervertebral implant between two vertebral bodies.
- Fig. 11: shows a perspective top view of the insert according to a second embodiment of the intervertebral implant
- Fig. 12: shows a top view of the intervertebral implant according to the second embodiment with a tool connected to the intervertebral implant in a first position.
- Fig. 13: shows a top view of the intervertebral implant according to the second embodiment with the tool connected thereto in a second position.
- Fig. 14: shows a perspective top view of the intervertebral implant according to a third embodiment with an insert not yet mounted.
- Fig. 15: shows a top view of the intervertebral implant of Fig. 14 according to the third embodiment with the insert mounted and a tool connected to the intervertebral implant in a first condition.
- Fig. 16: shows the intervertebral implant with the tool according to Fig. 15 in a second condition.
- Fig. 17: shows a perspective top view of the insert of an intervertebral implant according to a fourth embodiment.
- Fig. 18: shows a perspective bottom view of the insert of the intervertebral implant according to the fourth embodiment.
- Fig. 19: shows a top view of the intervertebral implant with insert according to the fourth embodiment and the tool connected thereto in a first position.
- Fig. 20: shows a top view of the intervertebral implant with the tool according to Fig. 19 in a second position.

Fig. 21 shows a perspective view from a top of an intervertebral implant with insert according to a fifth embodiment, and the tool connected thereto in a first position;
Fig. 22 shows a perspective view of an insert corresponding to the intervertebral implant according to the fifth embodiment;
Fig. 23 shows another perspective view of the insert corresponding to the intervertebral implant according to the fifth embodiment;
Fig. 24 shows a perspective view of the intervertebral implant without the insert according to the fifth embodiment; and
Fig. 25 shows another perspective view of the intervertebral implant without the insert according to the fifth embodiment.

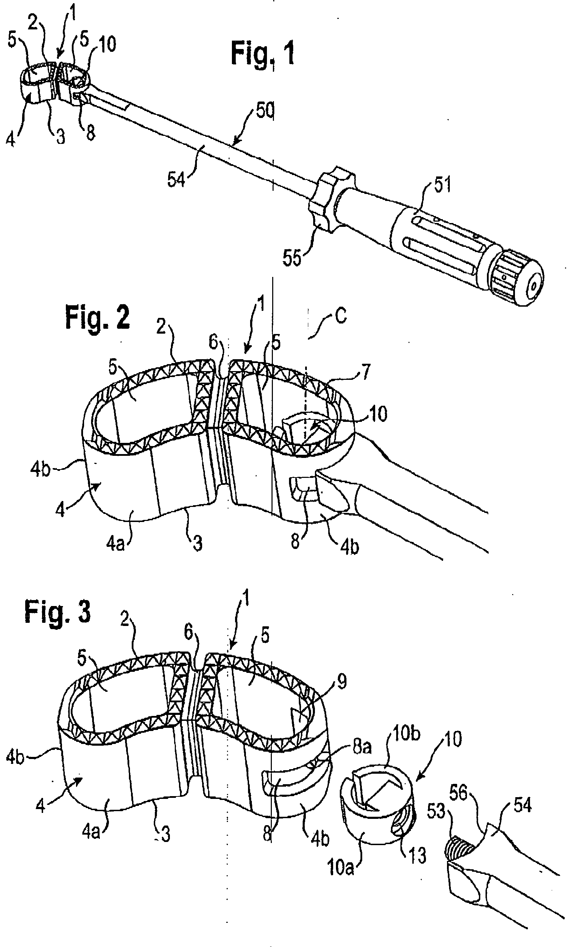

Fig. 1 shows a perspective view of a first embodiment of an intervertebral implant 1 and a tool 50 for inserting the intervertebral implant 1. As shown in particular in Figs. 1 to 3, the intervertebral implant includes a top face 2, a bottom, face 3 and a sidewall 4 connecting the top face 2 and the bottom face 3. The sidewall 4 defines an interior hollow section 5. The top face 2 and the bottom face 3 have openings so that the hollow interior section 5 extends into the top face 2 and the bottom face 3. Hence, the top face 2 and the bottom face 3 are formed by the upper and lower rim of the sidewall 4. Furthermore, a center wall 6 may be provided that separates the hollow interior section 5 in two parts. The height of the sidewall 4 is such that the implant can be inserted between a first and a second vertebral body. The height of the implant may be largest around the center wall 6 and may decrease towards the outer ends. Teeth 7 or other engagement portions are projecting from the top face 2 and the bottom face 3 for engaging the end plates of the vertebral bodies.

The implant 1 has two opposite long sides 4a and two opposite short sides 4b connecting the long sides 4a. The short sides 4b are rounded. The contour of the sidewall 4 may be arcuate, for example, it may have a kidney-shape or a banana-shape.

As can be seen in particular in Figs. 2 and 3, an opening 8 is provided in the sidewall 4 that extends completely through the sidewall 4 into the interior hollow section 5. The opening 8 has an elongate shape and extends preferably over a length in a circumferential direction along a short side 4b. The opening 8 may have a substantially rectangular contour and is located substantially in the center of the sidewall in a vertical direction between the top face 2 and the bottom face 3.

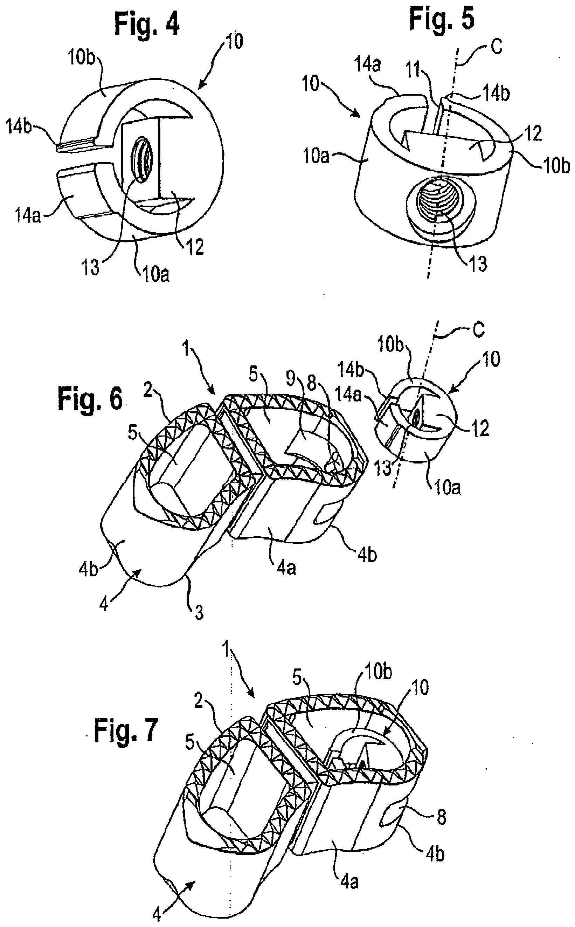

As shown in Fig. 6, a guide groove 9 is provided at the inner wall of the sidewall 4 around the elongate opening 8. The guide groove 9 extends from both ends of the elongate opening into each of the long sides 4a of the sidewall. The height of the guide groove 9 in the vertical direction, i. e. in the direction from the bottom face 3 to the top face 2 is the same or greater than the height of the elongate opening 8 in a vertical direction. The length of the guide groove 9 in a circumferential direction is dimensioned such that an insert 10 shown in Figs. 1 to 7 can be accommodated and guided in the groove. When the insert 10 is inserted into the hollow interior section 5 and placed into the groove 9, the groove 9 prevents falling out of the insert 10.

The insert 10 of the implant 1 according to the first embodiment is a substantially hollow cylindrical member. The cylinder axis C defines an axis of rotation. A coaxial slot 11 through the wall of the hollow cylinder provides first and second flexible substantially semi-spherical arms 10a, 10b that can be compressed slightly towards each other thereby narrowing the slot 11. At a position opposite to the slot 11, the insert 10 comprises a solid portion 12, for example, a cuboid-like portion extending between the flexible arms 10a, 10b. The size of the solid portion 12 is such that the flexibility of the arms 10a, 10b is not restricted. The solid portion 12 has a recess 13 for engagement with the tool 50. The recess 13 can be, for example, a threaded bore.

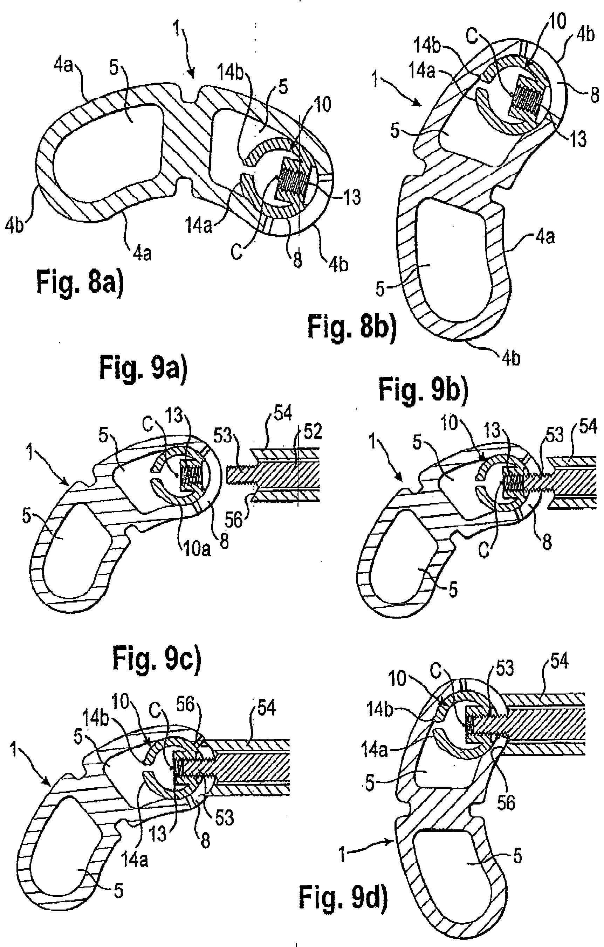

The first flexible arm 10a has at its outer wall adjacent the free end a first stop in form of a first protrusion 14a. The second flexible arm 10b has at its outer wall adjacent the free end a second stop in form of a second protrusion 14b. As shown in Figs. 8a and 8b, the insert 10 provides with its cylinder axis C an axis of rotation for the implant. Hence, when the insert 10 is inserted, it can be rotated relative to the implant between the first position defined by the abutment of the first protrusion 14a at the inner wall (Fig. 8a) and a second position defined by the abutment of the second stop 14b at the inner wall (Fig. 8b). The first protrusion 14a is at a position away from the slot 11 such that when the insert 10 is within the guide groove 9 and the first protrusion 14a abuts against the inner wall of the sidewall of the implant, recess 13 is located at the end of the opening 8 opposite to the first protrusion 14a. The second protrusion 14b is at a position at the free end of the second flexible arm 10b such that the recess 13 is at the other end of the elongate opening 8 when the protrusion 14b abuts against the opposite inner wall of the sidewall 4 of the implant 1.

The outer radius of the insert 10 corresponds substantially to the radius of the guide groove. The overall size of the insert is such that when the flexible arms 10a, 10b are slightly compressed, the insert 10 can be inserted and rests in the guide groove 9 of the implant. The insert 10 is guided by the guide groove 9 and held by the frictional force between the flexible arms 10a, 10b and the inner wall of the implant.

The tool 50 will now be explained with reference to Figs. 1 to 8. It comprises a handle 51 that is connected to a drive shaft 52 with an end portion 53 for engagement with the recess 13. In the embodiment shown, the end portion 53 is a threaded end portion that engages the threaded bore. The drive shaft 52 is rotatable within a sleeve 54 that is connected to a counter holding portion 55. An end portion 56 of the sleeve is concavely curved with a curvature adapted to the curvature of the outer wall of the short side 4b of the side wall 4. The tool is, however, not limited to the specific example shown. It may have a different construction.

The connection between the tool and the implant will now be explained with reference to Figs. 9a) to 9d). When the insert 10 is inserted into the implant as shown in Fig. 9a), the recess 13 faces the opening 8. The recess 13 is engaged with the end portion 53 of the drive shaft 52. In the case of a threaded bore and a threaded end portion, screwing the end portion into the bote results in clamping the short side 4b of the implant between the insert and the end portion 56 of the sleeve 54 of the tool 50. The connection between the implant and the tool is fixed. A slight loosening of the threaded connection allows a rotation of the insert with tool relative to the implant. The rotation is limited in both directions by the abutment of the protrusions 14a, 14b, respectively, at the side wall 4. :

The implant 1 including the insert is made of a biocompatible material. For example, the implant can be made of stainless steel or titanium or a biocompatible metal alloy, such as a nickel titanium alloy, for example Nitinol, or can be made of a biocompatible plastic material, such as, for example, PEEK (polyetheretherketone).

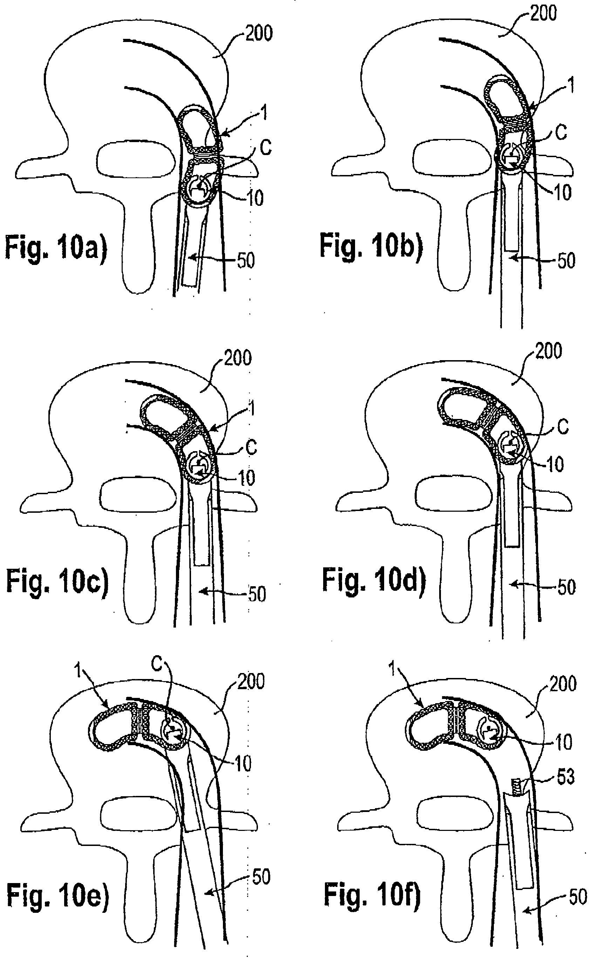

Use of the implant during surgery will now be described with reference to Figs. 10a) to 10f). Figs. 10a) to 10f) schematically show steps for inserting and positioning the intervertebral implant into the space between two vertebral bodies of adjacent vertebrae. First, the tool 50 is connected to the implant 1. The insert 10 is in such a position that the recess 13 is positioned at approximately the center of the elongate opening 8 in a lengthwise direction of the opening 8. By tightening the connection between the insert and the tool, the implant 1 is fixed to the tool 50. Then, as shown in Fig. 10a), the implant 1 is introduced into the intervertebral space between two neighbouring vertebrae, one of them is shown as vertebra 100 in the drawings. The narrow side 4b of the implant that is opposite to the tool 50 is the leading side. In the method shown, the intervertebral implant 1 is introduced into the space between the vertebral bodies using a posterior and lateral approach to access the space between the vertebral bodies.

As soon as the implant 1 and the tool 50 experience resistance and cannot be pushed further, as shown in Fig. 10b), the fixation between the implant 1 and the tool is loosened slightly by screwing back the drive shaft. This loosens the clamping so that the implant 1 is rotatable about the cylinder axis C of the insert 10. As shown in Figs. 10c) and 10d), the implant 1 rotates around the cylinder axis of the insert 10 so that the elongate opening 8 moves along the recess 13 of the insert 10. Then, as shown in Fig. 10e), the implant is shifted to its final position. As depicted in Fig. 10f), thereafter, the tool 50 is disconnected by unscrewing the drive shaft from the threaded recess 13 and removed.

Since the tool can be easily connected to and separated from the implant, the handling or the implant is simplified. During implantation, the implant is safely connected to the tool and cannot escape.

Furthermore, the design of the insert allows, that most of the hollow interior space is available used for fusion. The insert 10 remains with the implant and occupies very little space therein.

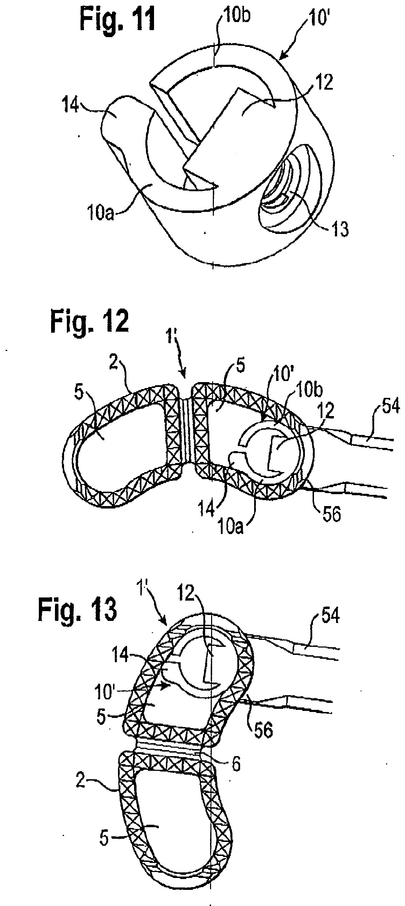

Figs. 11 to 13 illustrate a second embodiment of the implant. The implant 1' differs from the implant 1 of the first embodiment by the design of the insert. The insert 10' of the second embodiment differs from the insert 10 of the first embodiment by the construction of the stop. All other parts of the implant and the insert are the same as for the first embodiment. The description thereof will not be repeated. The insert 10' has instead of two separate stops provided at the flexible arms, respectively, only one single protrusion 14 that is formed by a thickened end portion of the first flexible arm 10a. The thickened end portion 14 extends along the axial length of the insert 10' and is provided adjacent to the slot 11. By the thickened end portion 14 the outer diameter at the end of the flexible arm 10a is greater than the outer diameter at the flexible arm 10b. The size of the thickened end portion is such that it can limit the rotation of the implant between two positions as shown in Figs. 12 and 13. When the insert 10' is in a first position, the recess 13 of the insert is approximately at the center of the elongate opening 8 in a lengthwise direction of the opening. The thickened portion 14 abuts against one of the long sides 4a of the sidewall 4. The connection between the tool 50 and the implant is substantially straight as shown in Fig. 12. When the insert 10' is rotated relative to the implant 1' by approximately 90°, the thickened end portion 14 abuts against the opposite of the long sides 4a of the sidewall and the recess 13 of the insert 10' is at one end of the elongate opening opposite to the thickened portion 14. The connection between the tool and the implant 1' is substantially perpendicular as shown in Fig.13.

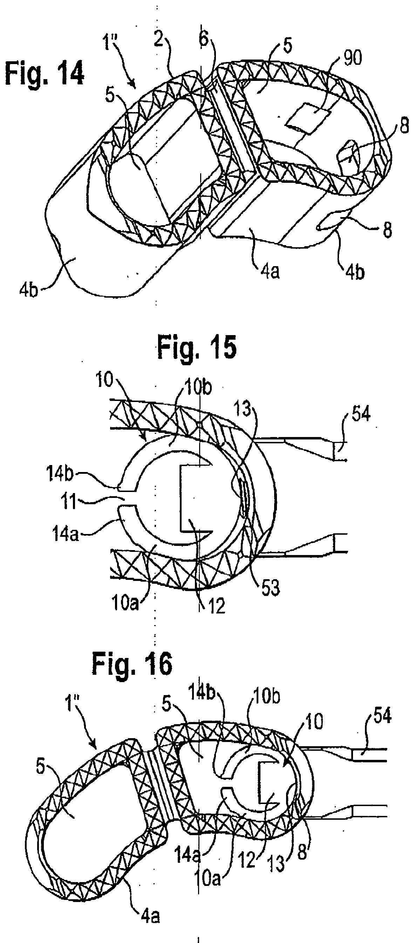

A third embodiment of the implant is illustrated in Figs. 14 to 16. The implant 1" differs from the implant according to the first embodiment by the position of the guide groove. The guide groove 90 consists of two groove parts located opposite to each other at the inner wall of the sidewall 4 in the area of the long sides 4a. The groove parts of the guide groove 90 are located at a distance to the elongate opening 8. When the insert 10 is inserted into the hollow interior section 5, a portion of the flexible arms 10a, 10b is received in the guide groove 90, respectively. As can be seen in Fig. 15, the position of the guide groove 90 is such that when the insert is inserted, there is a distance between the recess 13 and the opening 8.

Fig. 15 shows the engagement of the end portion 53 of the drive shaft 52 of the tool 50 with the recess 13 of the insert 10. Since there is a distance between the insert and the opening 8, the connection is not fixed and the implant can be rotated around the insert 10. By further engagement of the tool and the insert, the insert is drawn against the opening by slightly compressing the flexible arms 10a, 10b so that the connection between the implant and the tool is fixed.

The implant 1" can be equipped also with the insert 10' according to Figs. 11 to 13.

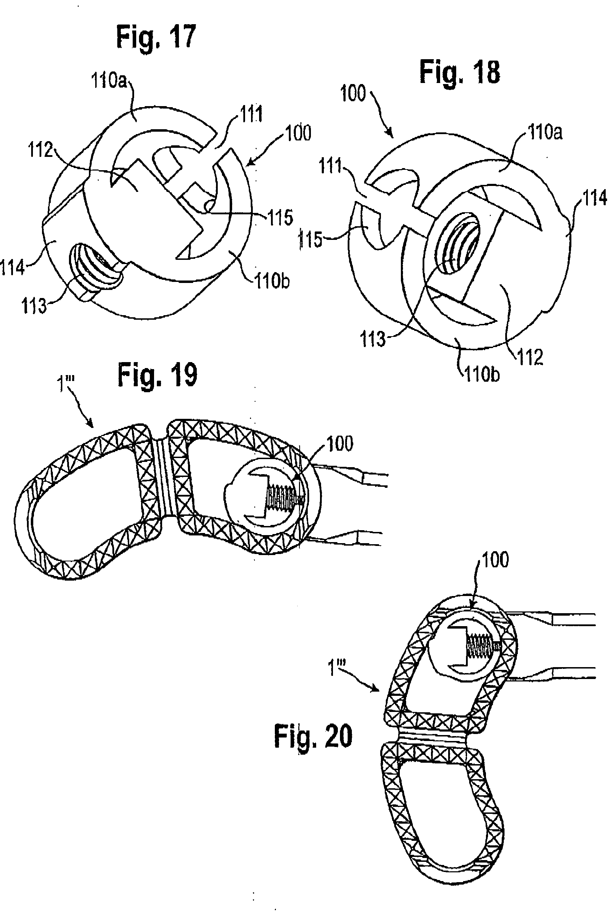

A fourth embodiment of the implant is shown in Figs. 17 to 20. The implant 1"' according to the fourth embodiment comprises an insert 100 that is different from the inserts 10 and 10' of the previous embodiments in that it can be mounted such that the flexible portions of the insert are facing towards the elongate opening. The insert 100 is a substantially hollow cylindrical part as in the previous embodiments, that has a slot 111 providing two flexible arms 110a, 110b. Opposite to the slot 111, a solid portion 112 extends into the space between the flexible arms 110a, 110b. The solid portion 112 has an engagement portion 113 for the tool which can be a threaded through hole. At the outer side that faces away from the slot 111, the insert 100 comprises a protrusion 114 that extends from the threaded through hole 113 asymmetrically to the side of the first flexible arm 110a. The protrusion 114 acts as a stop to limit the rotation of the insert 100 relative to the implant in two directions. The insert 100 further comprises a circular recess 115 that is provided at the position of the slot to allow the end portion 53 of the tool 50 to be guided there through.

Figs. 19 and 20 show atop view of the implant 1"' with the insert 100 mounted therein. The insert 100 is mounted such that the flexible arms 110 a, 110b are oriented towards the opening 8. Hence, the threaded through hole 113 has a distance from the opening 8. The end portion 53 of the drive shaft 52 of the tool 50 is guided through the elongate opening 8 and engages the threaded through hole 113. The size of the circular recess 115 is such that the end portion 53 can pass therethrough. When the threaded connection between the end portion 53 and the through hole 113 is tightened, the flexible arms 110a, 110b are slightly compressed and drawn against the opening 8. Thereby, the connection between the implant and the tool is fixed. Loosening the connection allows a rotation of the implant. As shown in Fig. 19, the movement of the implant relative to the tool is limited in a first direction when the protrusion 114 abuts against one of the long sides 4a of the sidewall. In this position, the threaded through hole 113 points to approximately the center of the elongate opening 8. When the implant is rotated with respect to the tool, the other portion of the protrusion 114 abuts against the other one of the long sides 4a of the side wall 4 to limit the rotational motion in the second direction. The range of motion is around 90°.

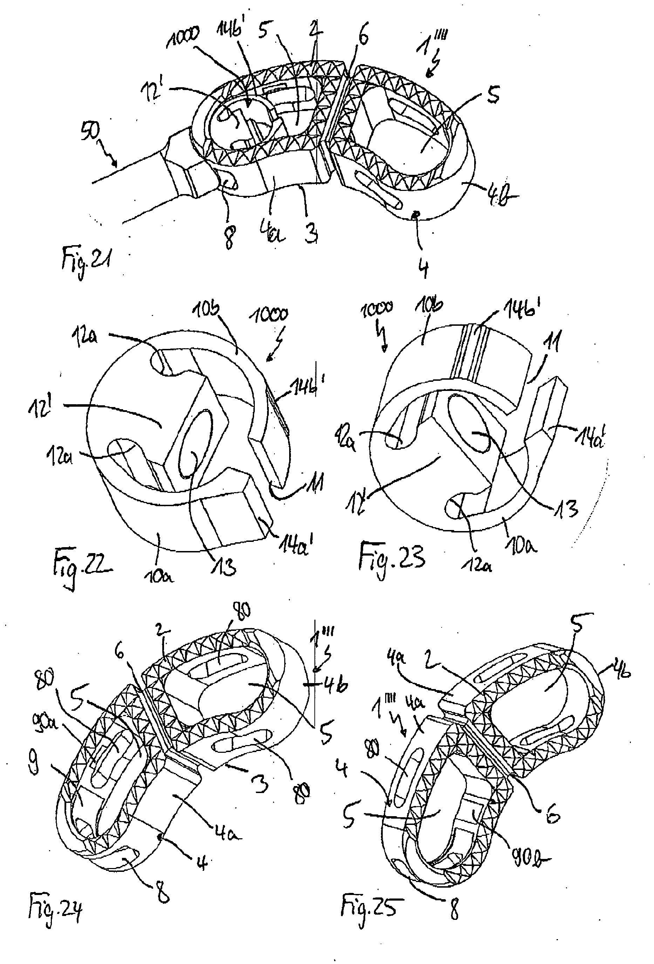

A fifth embodiment of the implant is shown in Figs. 21 to 25. Parts and portions that are identical or similar to the previous embodiments are indicated with the same reference numerals, and the descriptions thereof are therefore not repeated. In this embodiment, a protrusion 14b' on insert 1000 is positioned on arm 10b at a position away from slot 11 in a circumferential direction. A position of the protrusion 14b' is selected such that when the insert 1000 is rotated relative to the implant into an end position similar to that shown in Fig. 8b, the protrusion 14b' snaps into groove 9. This may give feedback to a surgeon or other practitioner that such an end position has been reached.

Furthermore, side walls of solid portion 12' that face inner walls of flexible arms 10a, 10b each have a curved recess 12a to facilitate insertion of the insert 1000 between inner walls of implant 1"".

The implant 1"" may further have elongate openings 80 in sidewall 4 and additional grooves 90a, 90b. In particular, on a long side 4a, there may be additional elongate openings 80, for example, to allow ingrowth of bone material and vessels. Meanwhile, the grooves 90a, 90b at the long sides 4a may serve for easier insertion of the insert 1000 into interior hollow section 5. In such embodiments, once the insert 1000 has been inserted into the interior hollow section 5 at the position of the grooves 90a, 90b, the insert 1000 may then be displaced relative to the rest of the implant 1"" into the groove 9.

Modifications of the embodiments are possible. The contour and the shape of the implant may be different from the example shown. For example, the contour may have any other shape, it may be circular, rectangular, or oval etc. The height of the sidewall 4 may be constant throughout the implant. The center wall can be omitted. It is also possible to adapt existing intervertebral implants without such an opening by providing it with an elongate opening and a guide groove and the insert piece as described above.

The insert does not have to have the stops. When the tool 50 is connected to the implant, an abutment of the end portion 53 of the tool at the ends of the elongate opening 8 provides for a limitation of the relative movement. However, the stops are useful to limit the motion of the insert once it is inserted into the implant when the tool is not yet connected. The constructions of the various embodiments can be interchanged. For example, it is possible to have the design with the two groove portions 90 in any of the embodiments shown. The position and shape of the protrusions that act as a stop can vary. In particular, the shape can be different. The elongate opening 8 can be provided at another position and/or several openings can be provided.

The connection between the insert and the tool needs not to be a threaded connection. It can be any other connection that can be easily fixed and loosened, for example a snap-in connection.

## Claims

1. Intervertebral implant having
a top surface (2) configured to engage a first vertebral body,
a bottom surface (3) configured to engage a second vertebral body and
a side wall (4) connecting the top surface (2) and the bottom surface (3),
a hollow space (5) defined by the side wall and an elongate opening (8) extending through the side wall (4) into the space, the elongate opening (8) having a length in a circumferential direction;
the implant further comprising
a rotatable insert (10, 10', 100) provided within the space wherein the insert (10, 10', 100) comprises a spring portion (10a, 10b, 110a, 110b) that holds the insert within the space by frictional engagement with the side wall (4) and wherein the insert comprises an engagement portion (13, 113) that is configured to engage with a tool (50) through the opening,
whereby
the insert (10, 10', 100) is substantially cylindrical with a slot (11, 111) extending through the wall in an axial direction.

2. The intervertebral implant of claim 1, wherein the spring portion comprises two flexible arms (10a, 10b, 110a, 110b) engaging opposite inner wall portions of the side wall (4).

3. The intervertebral implant of claim 1 or 2, wherein the insert (10, 10', 100) provides an axis of rotation (C) for the intervertebral implant, the axis of rotation extending through the top surface (2) and the bottom surface (3).

4. The intervertebral implant of one of claims 1 to 3 wherein the insert (10, 10', 100) is guided in a guide groove (9, 90).

5. The intervertebral implant of claim 4, wherein the guide groove (9, 90) is provided at an inner wall portion of the side wall (4).

6. The intervertebral implant of claim 4 or 5, wherein the guide groove (9) is provided around the opening.

7. The intervertebral implant of one claims 4 to 6, wherein the guide groove (90) comprises two guide groove portions arranged substantially opposite to each other.

8. The intervertebral implant of one of claims 1 to 7, wherein the engagement portion is an opening (13) with an engagement structure for the tool, the opening facing away from the slot (11).

9. The intervertebral implant of one of claims 1 to 7, wherein the engagement portion is an opening (113) with an engagement structure for the tool, the opening facing towards the slot (111).

10. The intervertebral implant of one of claims 1 to 9, wherein at least one stop (14a, 14b, 14, 114) is provided that limits the rotation of the insert within the space.

11. The intervertebral implant of claim 10, wherein the at least one stop limits the rotation of the implant relative to the insert to around 90°.

12. The intervertebral implant of one of claims 1 to 10, wherein the side wall (4) defines a height of the implant and wherein the size of the insert (10, 10', 100) in the vertical direction is smaller than the height of the implant.

13. The intervertebral implant of one of claims 1 to 11, wherein the side wall (4) has a curvature along the elongate opening and wherein the rotatable insert (10, 10', 100) has an outer wall with a curvature corresponding to the curvature of the side wall.

14. The intervertebral implant of one of claims 1 to 13, wherein at least a second elongate opening extends through the side wall into the space.

15. The intervertebral implant of one of claims 1 to 14, wherein an inner wall of the side wall includes at least one groove to facilitate insertion of the insert into the space.

16. The intervertebral implant of one of claims 10 to 15, wherein one of the at least one stop is spaced apart from the slot.

## Patentansprüche

1. Zwischenwirbelimplantat mit
einer oberen Fläche (2), die ausgebildet ist in einen ersten Wirbelkörper einzugreifen
eine untere Fläche (3), die ausgebildet ist, in einen zweiten Wirbelkörper einzugreifen, und
eine Seitenwand (4), die die obere Fläche (2) und die untere Fläche (3) verbindet,
einen durch die Seitenwand definierten Hohlraum (5) und eine langgestreckte Öffnung (8), die sich durch die Seitenwand (4) in den Raum erstreckt, wobei die langgestreckte Öffnung (8) eine Länge in einer Umfangsrichtung hat;
wobei das Implantat ferner
einen innerhalb des Raums vorgesehenen drehbaren Einsatz (10, 10', 100) aufweist, wobei der Einsatz (10, 10', 100) einen federnden Abschnitt (10a, 10b, 110a, 110b) aufweist, der den Einsatz innerhalb des Raums durch einen Reibungseingriff mit der Seitenwand (4) hält und wobei der Einsatz einen Eingriffsabschnitt (13, 113) aufweist, der ausgebildet ist, durch die Öffnung mit einem Werkzeug (50) im Eingriff zu gelangen, wobei
der Einsatz (10, 10', 100) im Wesentlichen zylindrisch mit einem Schlitz (11, 111) ist, der sich in einer axialen Richtung durch die Wand erstreckt.

2. Zwischenwirbelimplantat nach Anspruch 1, wobei der federnde Abschnitt zwei flexible Arme (10a, 10b, 110a, 110b) aufweist, die mit gegenüberliegenden inneren Wandabschnitten der Seitenwand (4) im Eingriff sind.

3. Zwischenwirbelimplantat nach Anspruch 1 oder 2, wobei der Einsatz (10, 10', 100) eine Drehachse (C) für das Zwischenwirbelimplantat bereitstellt, wobei sich die Drehachse durch die obere Fläche (2) und die untere Fläche (3) erstreckt.

4. Zwischenwirbelimplantat nach einem der Ansprüche 1 bis 3, wobei der Einsatz (10, 10', 100) in einer Führungsnut (9, 90) geführt ist.

5. Zwischenwirbelimplantat nach Anspruch 4, wobei die Führungsnut (9, 90) an einem inneren Wandabschnitt der Seitenwand (4) vorgesehen ist.

6. Zwischenwirbelimplantat nach Anspruch 4 oder 5, wobei die Führungsnut (9) um die Öffnung herum vorgesehen ist.

7. Zwischenwirbelimplantat nach einem der Ansprüche 4 bis 6, wobei die Führungsnut (90) zwei Führungsnutabschnitte aufweist, die im Wesentlichen zueinander gegenüberliegend angeordnet sind.

8. Zwischenwirbelimplantat nach einem der Ansprüche 1 bis 7, wobei der Eingriffsabschnitt eine Öffnung (13) mit einer Eingriffsstruktur für das Werkzeug ist, wobei die Öffnung von dem Schlitz (11) weg zeigt.

9. Zwischenwirbelimplantat nach einem der Ansprüche 1 bis 7, wobei der Eingriffsabschnitt eine Öffnung (113) mit einer Eingriffsstruktur für das Werkzeug ist, wobei die Öffnung zu dem Schlitz (11) hin zeigt.

10. Zwischenwirbelimplantat nach einem der Ansprüche 1 bis 9, wobei zumindest ein Anschlag (14a, 14b, 14, 114) vorgesehen ist, der die Drehung des Einsatzes innerhalb des Raums begrenzt.

11. Zwischenwirbelimplantat nach Anspruch 10, wobei der zumindest eine Anschlag die Drehung des Implantats relativ zu dem Einsatz auf etwa 90° begrenzt.

12. Zwischenwirbelimplantat nach einem der Ansprüche 1 bis 10, wobei die Seitenwand (4) eine Höhe des Implantats definiert, und wobei die Größe des Einsatzes (10, 10', 100) in der vertikalen Richtung kleiner als die Höhe des Implantats ist.

13. Zwischenwirbelimplantat nach einem der Ansprüche 1 bis 11, wobei die Seitenwand (4) eine Krümmung entlang der langgestreckten Öffnung hat, und wobei der drehbare Einsatz (10, 10', 100) eine äußere Wand mit einer Krümmung hat, die der Krümmung der Seitenwand entspricht.

14. Zwischenwirbelimplantat nach einem der Ansprüche 1 bis 13, wobei sich zumindest eine zweite langgestreckte Öffnung durch die Seitenwand in den Raum erstreckt.

15. Zwischenwirbelimplantat nach einem der Ansprüche 1 bis 14, wobei eine innere Wand der Seitenwand zumindest eine Nut umfasst um das Einbringen des Einsatzes in den Raum zu erleichtern.

16. Zwischenwirbelimplantat nach einem der Ansprüche 10 bis 15, wobei einer des zumindest einen Anschlags von dem Schlitz beabstandet ist.

## Revendications

1. Implant intervertébral, présentant
une surface supérieure (2) configurée pour s'engager avec un premier corps vertébral,
une surface inférieure (3) configurée pour s'engager avec un deuxième corps vertébral, et
une paroi latérale (4) reliant la surface supérieure (2) et la surface inférieure (3),
un espace creux (5) défini par la paroi latérale et une ouverture allongée (8) s'étendant à travers la paroi latérale (4) dans l'espace, l'ouverture allongée (8) ayant une longueur dans une direction circonférentielle ;
l'implant comprenant en outre
un insert rotatif (10, 10', 100) prévu à l'intérieur de l'espace, l'insert (10, 10', 100) comprenant une partie de ressort (10a, 10b, 110a, 110b) retenant l'insert à l'intérieur de l'espace par engagement par friction avec la paroi latérale (4), et l'insert comprenant une partie d'engagement (13, 113) qui est configurée pour s'engager avec un outil (50) à travers l'ouverture, l'insert (10, 10', 100) étant substantiellement cylindrique avec une fente (11, 111) s'étendant à travers la paroi dans une direction axiale.

2. Implant intervertébral selon la revendication 1, dans lequel la partie de ressort comprend deux bras flexibles (10a, 10b, 110a, 110b) s'engageant avec des portions opposées de parois intérieures de la paroi latérale (4).

3. Implant intervertébral selon la revendication 1 ou 2, dans lequel l'insert (10, 10', 100) fournit un axe de rotation (C) pour l'implant intervertébral, l'axe de rotation s'étendant à travers la surface supérieure (2) et la surface inférieure (3).

4. Implant intervertébral selon l'une des revendications 1 à 3, dans lequel l'insert (10, 10', 100) est guidé dans une rainure de guidage (9, 90).

5. Implant intervertébral selon la revendication 4, dans lequel la rainure de guidage (9, 90) est prévue au niveau d'une portion de paroi intérieure de la paroi latérale (4).

6. Implant intervertébral selon la revendication 4 ou 5, dans lequel la rainure de guidage (9) est prévue autour de l'ouverture.

7. Implant intervertébral selon l'une des revendications 4 à 6, dans lequel la rainure de guidage (90) comprend deux parties de rainure de guidage disposées substantiellement à l'opposé l'une de l'autre.

8. Implant intervertébral selon l'une des revendications 1 à 7, dans lequel la partie d'engagement est une ouverture (13) avec une structure d'engagement pour l'outil, l'ouverture étant orientée dans la direction opposée de la fente (11).

9. Implant intervertébral selon l'une des revendications 1 à 7, dans lequel la partie d'engagement est une ouverture (113) avec une structure d'engagement pour l'outil, l'ouverture étant orientée dans la direction de la fente (111).

10. Implant intervertébral selon l'une des revendications 1 à 9, dans lequel au moins une butée (14a, 14b, 14, 114) est prévue, laquelle limite la rotation de l'insert à l'intérieur de l'espace.

11. Implant intervertébral selon la revendication 10, dans lequel l'au moins une butée limite la rotation de l'implant par rapport à l'insert à environ 90°.

12. Implant intervertébral selon l'une des revendications 1 à 10, dans lequel la paroi latérale (4) définit une hauteur de l'implant, et dans lequel la dimension de l'insert (10, 10', 100) dans la direction verticale est inférieure à la hauteur de l'implant.

13. Implant intervertébral selon l'une des revendications 1 à 11, dans lequel la paroi latérale (4) présente une courbure le long de l'ouverture allongée, et dans lequel l'insert rotatif (10, 10', 100) présente une paroi extérieure avec une courbure correspondant à la courbure de la paroi latérale.

14. Implant intervertébral selon l'une des revendications 1 à 13, dans lequel au moins une deuxième ouverture allongée s'étend à travers la paroi latérale dans l'espace.

15. Implant intervertébral selon l'une des revendications 1 à 14, dans lequel une paroi intérieure de la paroi latérale comporte au moins une rainure pour faciliter l'insertion de l'insert dans l'espace.

16. Implant intervertébral selon l'une des revendications 10 à 15, dans lequel l'une de l'au moins une butée est espacée de la fente.
